# EUROPÄISCHE PATENTSCHRIFT

(11) **EP 1 791 813 B1**
(45) Veröffentlichungstag und Bekanntmachung des Hinweises auf die Patenterteilung: **29.04.2009**
(21) Anmeldenummer: 05776339.3
(22) Anmeldetag: 27.08.2005
(51) Int. Cl.: C07C 381/00, A61K 31/155, A61P 7/04, A61P 9/06, A61P 25/24, A61P 33/06

(54) **PENTAFLUORSULFANYLPHENYL-SUBSTITUIERTE BENZOYLGUANIDINE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
PENTAFLUOROSULFANYLPHENYL-SUBSTITUTED BENZOYLGUANIDINES, METHOD FOR THE PRODUCTION THEREOF, THEIR USE AS A MEDICAMENT OR DIAGNOSTIC AGENT, AND A MEDICAMENT CONTAINING THESE COMPOUNDS
BENZOYLGUANIDINES SUBSTITUEES PAR PENTAFLUORSULFANYLPHENYLE, PROCEDE POUR LES PREPARER, LEUR UTILISATION EN TANT QUE MEDICAMENT OU QU'AGENT DIAGNOSTIQUE, ET MEDICAMENT LES CONTENANT

(30) Priorität: 11.09.2004 DE 102004043938
(43) Veröffentlichungstag der Anmeldung: 06.06.2007
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE)
(86) Internationale Anmeldenummer: PCT/EP2005/009272
(87) Internationale Veröffentlichungsnummer: WO 2006/027130

(56) Entgegenhaltungen:
- EP-A- 0 640 593
- EP-A- 0 723 956
- DE-A1- 10 222 192
- DE-A1- 10 226 462
- US-A1- 2005 043 401
- US-A1- 2005 124 666

## Beschreibung

Aus EP 0 640 593 A und EP 0 723 956 A sind bereits substituierte bzw. basischsubstituierte Benzoylguandine, die als pharmazeutische Wirkstoffe geeignet sind bekannt. Weiterhin werden in DE 102 26 462 A1 Cycloalkylsubstituierte-Benzoylgunadine offenbart. Darüber hinaus werden in DE 102 22 192 A1 Pentafluorosulfanylgruppen tragende Benzoylguanidine beschrieben, die sich allerdings von denen der vorliegenden Anmeldung strukturell unterscheiden. PentafluorosulfanylBenzoylguanidine, die sich strukturell von den hier beanspruchten unterscheiden werden auch in den Patentanmeldungen US 2005/124666 A1 und US 2005/043401 A1 offenbart, wobei beide Anmeldungen erst nach dem Prioritätstag der hier vorliegenden Anmeldung offengelegt wurden.

Die Erfindung betrifft Pentafluorsulfanylphenyl-substituierte Benzoylguanidine der Formel I 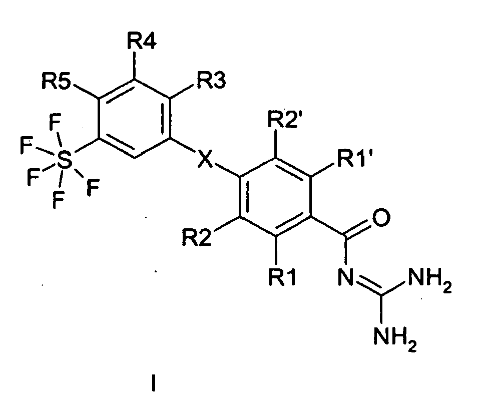 worin bedeuten
- R1 und R1': unabhängig voneinander Wasserstoff, Methyl, F, Cl, -CF₃ oder -O-CH₂-CF₃;
- R2 und R2': unabhängig voneinander Wasserstoff, F, Cl, -SO₂CH₃, -SO₂-CF₃, -CF₃ oder Methyl;
- R3: Wasserstoff, F, Cl oder Methyl;
- R4: Wasserstoff, F, Cl oder Methyl;
- R5: -SO₂CH₃;
- X: O;
sowie deren pharmazeutisch verträgliche Salze.

In einer Ausführungsform sind dabei Verbindungen der Formel I bevorzugt, in denen R1 und R1' unabhängig voneinander durch Wasserstoff, Methyl, F, Cl, -CF₃ oder -O-CH₂-CF₃ beschrieben werden, besonders bevorzugt sind Verbindungen, in denen R1 und R1' unabhängig voneinander durch Wasserstoff oder Methyl beschrieben werden.
In einer weiteren Ausführungsform sind Verbindungen der Formel I bevorzugt, in denen R2 und R2' unabhängig voneinander durch Wasserstoff, F, Cl, -SO₂CH₃, -SO₂CF₃, -CF₃ oder Methyl beschrieben werden, besonders bevorzugt sind Verbindungen, in denen R2 und R2' unabhängig voneinander durch Wasserstoff oder - SO₂CH₃ beschrieben werden.
In einer weiteren Ausführungsform sind Verbindungen der Formel I bevorzugt, in denen R3 durch Wasserstoff, F, Cl, oder Methyl beschrieben wird, besonders bevorzugt sind Verbindungen, in denen R3 durch Wasserstoff beschrieben wird.
In einer weiteren Ausführungsform sind Verbindungen der Formel I bevorzugt, in denen R4 durch Wasserstoff, F, Cl oder Methyl beschrieben wird, besonders bevorzugt sind Verbindungen, in denen R4 durch Wasserstoff beschrieben wird.
In einer weiteren Ausführungsform sind Verbindungen der Formel I bevorzugt, in denen R5 durch -SO₂CH₃ beschrieben wird.
In einer weiteren Ausführungsform sind Verbindungen der Formel I bevorzugt in denen X durch O beschrieben wird.

Reste, die mehrfach vorkommen, können gleich oder verschieden sein und unabhängig voneinander die angegebenen Bedeutungen haben.

Speziell bevorzugt sind N-[5-Methansulfonyl-4-(4-methansulfonyl-3-pentafluor-sulfanylphenoxy)-2-methyl-benzoyl]-guanidin und dessen pharmazeutisch verträglichen Salze.

Enthalten die Substituenten R1, R1', R2, R2', R3, R4 oder R5 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben in allen Verhältnissen vorliegen.

Die vorliegende Erfindung umfasst alle tautomeren Formen der Verbindungen der Formel 1.

Gegenstand der Erfindung sind auch die im folgenden beschriebenen Verfahren zur Herstellung der Verbindungen der Formel 1.

Die vorliegende Erfindung betrifft ein Verfahren zur Herstellung einer Verbindung der Formel I und/oder deren pharmazeutisch verträgliche Salze, worin X Sauerstoff ist (Schema 1), **dadurch gekennzeichnet, dass** man
a) ein Phenol der Formel III mit einem Aromaten der Formel IV zu einer Verbindung der Formel V umsetzt
b) durch elektrophile aromatische Substitution R5 einführt und zu einer Verbindung der Formel VI umsetzt
   und
c) eine Verbindung der Formel VI mit Guanidin zum Acylguanidin der Formel Ia umsetzt, 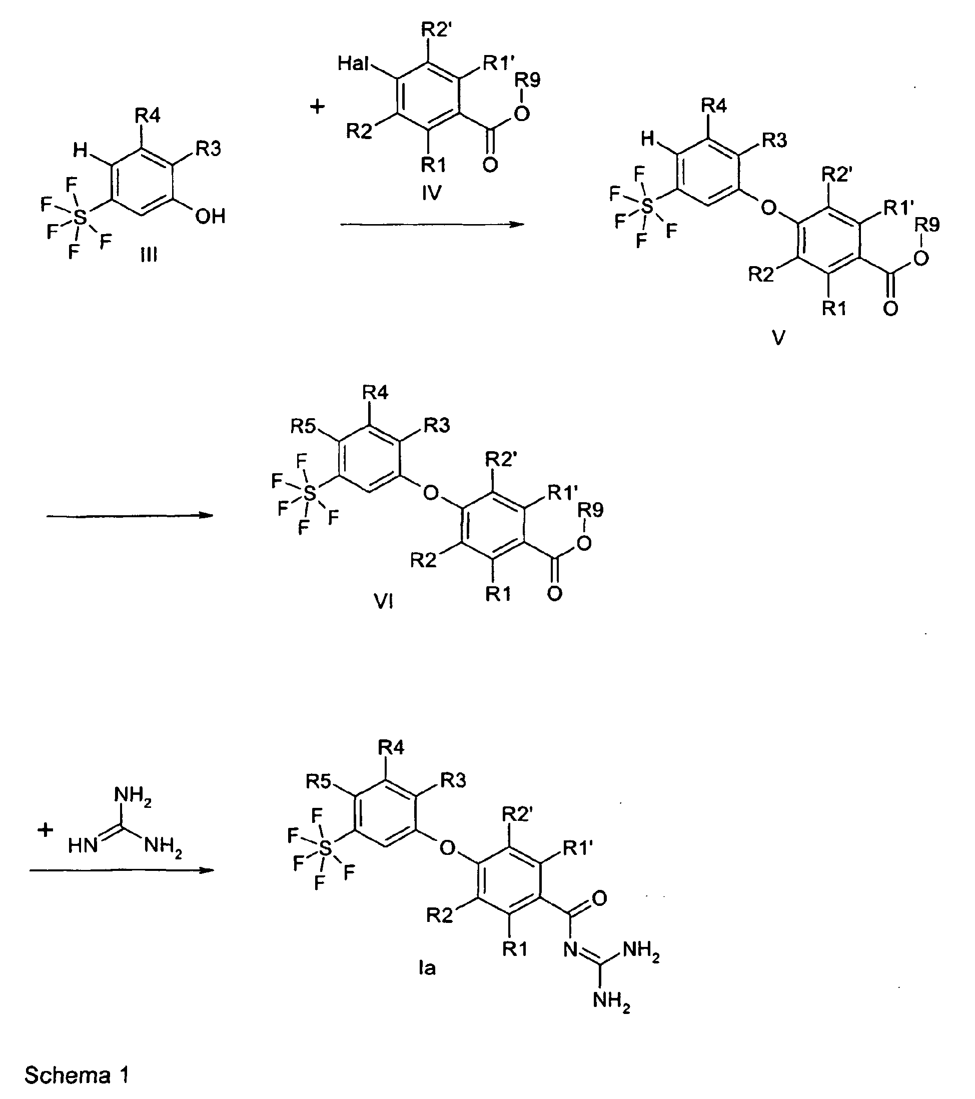
worin R1, R1', R2, R2', R3, R4 und R5 die oben angegebene Bedeutung besitzen und worin bedeuten
- Hal: F, Cl, Br oder I,
- R9: Wasserstoff oder Alkyl mit 1, 2, 3 oder 4 C-Atomen.

Die Phenole der Formeln III werden in einem geeigneten Lösungsmittel, bevorzugt in einem dipolar aprotischen Lösungsmittel wie zum Beispiel Acetonitril, DMF, NMP oder DMSO, mit Hilfe einer anorganischen Base, wie zum Beispiel K₂CO₃ oder Cs₂CO₃, oder mit Hilfe einer organischen Base, wie zum Beispiel Triethylamin oder TBTMG, bei einer Temperatur zwischen 0°C und dem Siedepunkt des verwendeten Lösungsmittels deprotoniert und dann mit dem elektrophilen Aromaten der Formel IV in einer nucleophilen aromatischen Substitution bei einer Temperatur zwischen 0°C und dem Siedepunkt des verwendeten Lösungsmittels, bevorzugt zwischen RT und 150°C, zu Verbindungen der Formel V umgesetzt.
Die Umsetzung der Verbindungen der Formel V zu den Verbindungen der Formel VI erfolgt durch elektrophile aromatische Substitution, bevorzugt durch Nitrierung, wie beispielsweise in Houben-Weyl, Methoden der organischen Chemie 4. Auflage, Organo-Stickstoff-Verbindungen IV, Teil 1, Georg Thieme Verlag Stuttgart 1992, S. 262-341 und in der dort zitierten Literatur beschrieben. Bevorzugt wird mit 90% HNO₃ bei einer Temperatur zwischen -80°C und RT, besonders bevorzugt zwischen -60°C und 0°C nitriert.
Aus den Verbindungen der Formel VI mit R5 = NO₂ können die entsprechenden Aniline (R5 = NH₂) wie in R.C. Larock, Comprehensive Organic Transformations: A Guide to Functional Group Preparations, VCH Publishers, New York, Weinheim, 1999, 821-828 und der dort zitierten Literatur beschrieben hergestellt werden. Aus diesen Anilinen werden über die Diazoniumsalze nach dem Fachmann bekannter Methode, wie zum Beispiel in Houben-Weyl, Methoden der organischen Chemie 4. Auflage, Organo-Stickstoff-Verbindungen I, Teil 2, Georg Thieme Verlag Stuttgart 1990, S. 1060-1136 sowie in den dort zitierten Literaturstellen beschrieben, die Verbindungen der Formel VI mit weiteren Bedeutungen von R5 synthetisiert.
Die Umsetzung der Verbindungen der Formel VI zu den Acylguanidinen der Formel Ia erfolgt entweder mit freier Guanidin-Base oder bevorzugt mit Guanidinium-Chlorid, das zunächst zusammen mit KOtBu in einem inerten Lösungsmittel, bevorzugt DMF oder NMP gerührt wird und dann zusammen mit dem Ester bei einer Temperatur zwischen 0°C und dem Siedepunkt des Lösungsmittels, bevorzugt zwischen RT und 100°C, gerührt wird.
Die Ester der Formel V in denen R9 Alkyl ist, können auch zunächst zu den Carbonsäuren verseift und anschließend, vorzugsweise in Gegenwart eines Aktivierungsmittels, mit Guanidin zu den Acylguanidinen der Formel Ia umgesetzt werden.
Die Ausgangsverbindungen der Formeln III und IV sind käuflich erhältlich oder können nach oder analog zu in der Literatur beschriebenen, dem Fachmann bekannten Verfahren hergestellt werden.

In den Ausgangsverbindungen können auch funktionelle Gruppen in geschützter Form oder in Form von Vorstufen vorliegen und dann in den nach dem oben beschriebenen Verfahren hergestellten Verbindungen der Formel I in die gewünschten Gruppen überführt werden. Entsprechende Schutzgruppentechniken sind dem Fachmann bekannt.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Zur Erfindung gehören auch Vorprodukte der Formel VI und deren Salze, 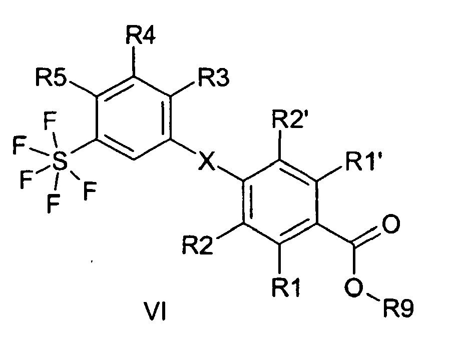 wobei R1, R1', R2, R2', R3, R4, R5 und X die oben genannten Bedeutungen haben und R9 Wasserstoff oder Alkyl mit 1, 2, 3 oder 4 C-Atomen ist, und ihre Verwendung als Syntheseintermediate, zum Beispiel zur Herstellung von Arzneimittelwirkstoffen wie zum Beispiel Verbindungen der Formel I und/oder deren pharmazeutisch verträgliche Salze durch Umsetzung mit Guanidin.

Pentafluorsulfanylphenyl-substituierte Benzoylguanidine der Formel I sind im allgemeinen schwache Basen und können Säure unter Bildung von Salzen binden. Als Säureadditionssalze kommen insbesondere Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen der Formel I sind substituierte Acylguanidine und inhibieren den zellulären Natrium-Protonen-Antiporter (Na⁺/H⁺-Exchanger, NHE), insbesondere den Subtyp NHE-1.

Gegenüber bekannten NHE-Inhibitoren zeichnen sich die erfindungsgemäßen Verbindungen durch eine außerordentlich hohe Wirksamkeit in der Inhibition des Na⁺/H⁺-Austauschs aus, sowie durch verbesserte ADMET-Eigenschaften aus, zum Beispiel durch längere S9-Stabilitäten (Leberstabilitäten, Stabilität gegenüber enzymatischem Angriff) und hoher Selektivität gegenüber dem hERG-Kaliumkanal. Dabei weisen sie ein gutes Resorptionsverhalten und eine hohe Bioverfügbarkeit auf.

Aufgrund der NHE-inhibitorischen Eigenschaften eignen sich die Verbindungen der Formel I und/oder deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHEbedingten Schädigungen verursacht werden.

Da NHE-Inhibitoren in überwiegender Weise über ihre Beeinflussung der zellulären pH-Regulation wirken, können diese generell in günstiger Weise mit anderen, den intrazellulären pH-Wert regulierenden Verbindungen kombiniert werden, wobei Inhibitoren der Enzymgruppe der Carboanhydrasen, Inhibitoren der Bicarbonationen transportierenden Systeme wie des Natrium-Bicarbonat-Cotransporters (NBC) oder des Natrium abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

So eignen sich die erfindungsgemäßen Inhibitoren des NHE zur Behandlung von Krankheiten, die durch Ischämie und durch Reperfusion hervorgerufen werden.

Die hier beschriebenen Verbindungen sind infolge ihrer pharmakologischen Eigenschaften als antiarrhythmische Arzneimittel geeignet.
Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I und/oder deren pharmazeutisch verträgliche Salze infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die erfindungsgemäßen Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden können die erfindungsgemäßen Verbindungen der Formel I auch bei Wiederbelebung nach einem Herzstillstand eingesetzt werden.

Die erfindungsgemäßen Verbindungen sind von Interesse für Arzneimittel gegen lebensbedrohliche Arrhythmien. Kammerflimmern wird beendet und der physiologische Sinus-Rhythmus des Herzens wiederhergestellt.

Da NHE1-Inhibitoren menschliches Gewebe und Organe, insbesondere das Herz, nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist die kombinierte Verabreichung mit Verbindungen der Formel I und/oder deren pharmazeutisch verträgliche Salze geeignet, die cytotoxischen, insbesondere cardiotoxischen Nebenwirkungen der genannten Verbindungen zu inhibieren. Durch die Verminderung der cytotoxischen Effekte, insbesondere der Cardiotoxizität, infolge Ko-Medikation mit NHE1-Inhibitoren kann außerdem die Dosis der cytotoxischen Therapeutika erhöht und/oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Außerdem können die erfindungsgemäßen NHE1-Inhibitoren der Formel I und/oder deren pharmazeutisch verträgliche Salze bei einer herzschädigenden Überproduktion von Schilddrüsenhormonen, der Thyreotoxikose, oder bei der externen Zufuhr von Schilddrüsenhormonen Verwendung finden. Die Verbindungen der Formel I und/oder deren pharmazeutisch verträgliche Salze eignen sich somit zur Verbesserung der Therapie mit cardiotoxischen Arzneimitteln.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie zum Beispiel zur Behandlung des Schlaganfalls oder des Hirnödems geeignet sind.

Die Verbindungen der Formel I und/oder deren pharmazeutisch verträgliche Salze eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydrasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäß verwendeten Verbindungen der Formel I und/oder deren pharmazeutisch verträgliche Salze ebenfalls zur Behandlung von Formen des Schocks, wie beispielweise des allergischen, cardiogenen, hypovolämischen und des bakteriellen Schocks.

Die Verbindungen der Formel I und/oder deren pharmazeutisch verträgliche Salze können ebenfalls zur Prävention und zur Behandlung thrombotischer Erkrankungen verwendet werden, da sie als NHE-Inhibitoren die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und der thrombogenen Selectin-Proteine hemmen bzw. verhindern. Damit kann die pathogene Wirkung bedeutender thrombogener Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan Rezeptor-Antagonisten, Phosphodiesterase-Inhibitoren, Factor-Vlla-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydrase, wie beispielsweise mit Acetazolamid, ist besonders günstig.

Darüber hinaus zeichnen sich NHE1-Inhibitoren durch starke inhibierende Wirkung auf die Proliferationen von Zellen, beispielsweise der Fibroblasten-Proliferation und der Proliferation der glatten Gefäßmuskelzellen, aus. Deshalb kommen die Verbindungen der Formel I und/oder deren pharmazeutisch verträgliche Salze als wertvolle Therapeutika für Krankheiten in Frage, bei denen die Proliferation eine primäre oder sekundäre Ursache darstellt, und können deshalb als Antiatherosklerotika, Mittel gegen das chronische Nierenversagen, Krebserkrankungen, verwendet werden.

Es konnte gezeigt werden, dass durch NHE-Inhibitoren die Zellmigration inhibiert wird. Daher kommen die Verbindungen der Formel I und/oder deren pharmazeutisch verträgliche Salze als wertvolle Therapeutika für Krankheiten in Frage, bei denen die Zellmigration eine primäre oder sekundäre Ursache darstellt, wie beispielsweise Krebserkrankungen mit ausgeprägter Neigung zur Metastasierung.

NHE1-Inhibitoren zeichnen sich weiterhin durch eine Verzögerung oder Verhinderung von fibrotischen Erkrankungen aus. Verbindungen der Formel I und/oder deren pharmazeutisch verträgliche Salze eignen sich somit als Mittel zur Behandlung von Fibrosen des Herzens, sowie der Lungenfibrose, Leberfibrose, der Nierenfibrose und anderer fibrotischer Erkrankungen. Sie können somit zur Behandlung von Organhypertrophien und -hyperplasien, beispielsweise des Herzens und der Prostata verwendet werden. Sie sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Da der NHE bei essentiellen Hypertonikern signifikant erhöht ist, eignen sich die Verbindungen der Formel I und/oder deren pharmazeutisch verträgliche Salze zur Prävention und zur Behandlung des Bluthochdrucks und zur Behandlung von Herz-Kreislauferkrankungen. Dabei können sie allein oder mit einem geeigneten Kombinations- und Formulierungspartner zur Bluthochdruckbehandlung und von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, kombiniert werden. Weiterhin können die NHE-Inhibitoren der vorliegende Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromakalim, Minoxidil und deren Derivate, Aktivatoren des mitochondrialen ATP-sensitiven Kaliumkanals (mitoK(ATP) Kanal), Inhibitoren des Kv1.5 usw.

Es zeigte sich, dass NHE1-Inhibitoren eine signifikante antiphlogistische Wirkung haben und somit als Antiinftammatorika verwendet werden können. Dabei fällt die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antünflammatorika verwendet. Weiterhin könne die erfindungsgemäßen Verbindungen zur Prävention oder Behandlung von Erkrankungen, die durch Protozoen verursacht werden, eingesetzt werden, wie bei Malaria und der Hühnercoccidiose.

Es wurde außerdem gefunden, dass NHE1-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, dass NHE1-Inhibitoren bezüglich der Beeinflussung der Serumlipidspiegel wertvolle therapeutisch verwertbare Eigenschaften zeigen. So erniedrigen sie signifikant die erhöhte SerumKonzentrationen von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie zum Beispiel beim Diabetes vorkommen. Darüber hinaus führen die NHE1-Inhibitoren zu einer deutlichen Reduktion der durch Stoffwechselanomalien induzierten Infarkte und insbesondere zu einer signifikanten Verminderung der induzierten Infarktgröße und dessen Schweregrades.

Die Verbindungen der Formel I und/oder deren pharmazeutisch verträgliche Salze finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und AngiotensinRezeptorantagonisten. Eine Kombination eines NHE-Inhibitors der Formel I und/oder deren pharmazeutisch verträgliche Salze mit einem blutfettspiegelsenkenden Wirkstoff, bevorzugt mit einem HMG-CoA-Reduktaseinhibitor (zum Beispiel Lovastatin oder Pravastatin), wobei letzterer eine hypolipidämische Wirkung herbeiführt und dadurch die hypolipidämischen Eigenschaften des NHE-Inhibitors der Formel I und/oder deren pharmazeutisch verträgliche Salze steigert, erweist sich als günstige Kombination mit verstärkter Wirkung und vermindertem Wirkstoffeinsatz.

So führen Verbindungen der Formel I und/oder deren pharmazeutisch verträgliche Salze zu einen wirksamen Schutz gegen Endothelschädigungen unterschiedlicher Genese. Mit diesem Schutz der Gefäße gegen das Syndrom der endothelialen Dysfunktion sind Verbindungen der Formel I und/oder deren pharmazeutisch verträgliche Salze wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem wurde gefunden, dass NHE1-Inhibitoren geeignet in der Behandlung des nicht-insulinabhängigen Diabetes (NIDDM) sind, wobei die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR Agonisten, wie Rosiglitazone, Pioglitazone etc., mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken die Verbindungen der Formel I und/oder deren pharmazeutisch verträgliche Salze der Entstehung diabetischer Spätkomplikationen entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den soeben unter NIDDM-Behandlung beschriebenen antidiabetischen Arzneimitteln vorteilhaft kombiniert werden. Der Kombination mit einer günstigen Darreichungsform von Insulin dürfte dabei eine besondere Bedeutung zukommen.

NHE1-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die unabhängig von akuten Mangeldurchblutungszuständen sind und bei normalen, nichtischämischen Bedingungen auftreten. Bei diesen pathologischen, über die lange Zeit des Altems ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. Die Verbindungen der Formel I und/oder deren pharmazeutisch verträgliche Salze eignen sich somit hervorragend zur Behandlung und Prävention der altersbedingt fortschreitenden endothelialen Dysfunktion, insbesondere von claudicatio intermittens.

Beispiel einer weiteren den Altersprozess charakterisierenden Messgröße ist die Abnahme der Kontraktilität des Herzens und die Abnahme der Anpassung des Herzens an eine geforderte Pumpleistung des Herzens. Diese verminderte Herzleistungsfähigkeit als Folge des Alterungsprozesses ist in den meisten Fällen verbunden mit einer Dysfunktion des Herzens, die unter anderem durch eine Einlagerung von Bindegewebe ins Herzgewebe verursacht wird. Diese Bindegewebseinlagerung ist gekennzeichnet durch eine Zunahme des Herzgewichtes, durch eine Vergrößerung des Herzens und durch eine eingeschränkte Herzfunktion. Es war überraschend, dass eine derartige Alterung des Organs Herz nahezu komplett inhibiert werden konnte. Die Verbindungen der Formel I und/oder deren pharmazeutisch verträgliche Salze eignen sich somit hervorragend zur Behandlung und Prävention der Herzinsuffizienz, des congestive heart failure (CHF).
Durch Proliferationshemmung können nicht nur die bereits eingetretene Krebserkrankung geheilt werden, sondern auch die altersbedingte Entstehungshäufigkeit von Krebs durch NHE-Inhibitoren vermindert und hochsignifikant verzögert werden. Besonders bemerkenswert ist der Befund, dass altersbedingt auftretenden Erkrankungen aller Organe und nicht nur bestimmter Krebsformen unterbunden bzw. hochsignifikant verzögert auftreten. Die Verbindungen der Formel I und/oder deren pharmazeutisch verträgliche Salze eignen sich somit zur Behandlung und insbesondere der Prävention von altersbedingten Formen von Krebs.

Mit NHE-Inhibitoren wird eine zeitlich hochsignifikant verschobene Verzögerung des Eintretens altersbedingter Erkrankungen aller untersuchten Organe einschließlich Herz, Gefäße, Leber usw., sowie eine hochsignifikante Verzögerung von Alterskrebs festgestellt. Vielmehr kommt es auch überraschenderweise zu einer Lebensverlängerung in einem Ausmaß, das bislang durch keine andere Medikamentengruppe bzw. durch irgendwelche Naturstoffe erreicht werden konnte. Diese einzigartige Wirkung der NHE-Inhibitoren ermöglicht es auch, neben der alleinigen Wirkstoffanwendung an Mensch und Tier diese NHE-Inhibitoren mit anderen gerontologisch verwendeten Wirkprinzipien, Maßnahmen, Substanzen und Naturstoffen zu kombinieren, denen ein anderer Wirkmechanismus zugrunde liegt. Derartige in der gerontologischen Therapie verwendete Wirkstoffklassen sind: insbesondere Vitamine und antioxidativ wirksame Stoffe. Da eine Korrelation zwischen kalorischer Belastung bzw. Nahrungsaufnahme und Alterungsprozeß besteht, kann die Kombination mit diätetischen Maßnahmen zum Beispiel mit Appetitszüglern erfolgen. Ebenso kann eine Kombination mit blutdrucksenkenden Medikamenten, wie mit ACE-Hemmern, Angiotensinrezeptorantagonisten, Diuretika, Ca²⁺-Antagonisten etc. oder mit stoffwechselnormalisierenden Medikamenten, wie Cholesterinsenkern, gedacht werden.

Die Verbindungen der Formel I und/oder deren pharmazeutisch verträgliche Salze eignen sich somit hervorragend zur Prävention altersbedingter Gewebsveränderungen und zur Lebensverlängerung unter Erhalt einer hohen Lebensqualität.
Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Beansprucht wird weiterhin ein Heilmittel für die humane, veterinäre oder phytoprotektive Anwendung enthaltend eine wirksame Menge einer Verbindung der Formel 1 und/oder deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.

Arzneimittel, die eine Verbindung der Formel I und/oder deren pharmazeutisch verträgliche Salze enthalten, können dabei zum Beispiel oral, parenteral, intravenös, rektal, perkutan oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, intramuskulären oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage: Wasser, physiologische Kochsalzlösung oder Alkohole, zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen des Wirkstoffes der Formel und/oder deren pharmazeutisch verträgliche Salze in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I und/oder deren pharmazeutisch verträgliche Salze bei einem etwa 75 kg schweren Patienten mindestens 0,001 mg/kg, beispielsweise 0,01 mg/kg, bis höchstens 10 mg/kg, beispielsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, zum Beispiel bis zu 4 Einzeldosen pro Tag. Insbesondere bei i.v. Anwendung, etwa bei einem Infarktpatienten auf der Intensivstation können beispielsweise bis zu 700 mg pro Tag notwendig werden und können die erfindungsgemäßen Verbindungen durch Infusion verabreicht werden.

### Liste der Abkürzungen:

- ADMET: Absorption - Distribution - Metabolismus - Ausscheidung - Toxikologie
- DIP: Diisopropylether
- DMF: N,N-Dimethylformamid
- DMSO: Dimethylsulfoxid
- EE: Ethylacetat
- HEP: n-Heptan
- hERG: human ether-a-go-go-related gene
- HOAc: Essigsäure
- KOtBu: Kalium-2-methyl-propan-2-olat
- MeOH: Methanol
- mp: Schmelzpunkt
- MTB: tert.-Butyl-methylether
- NMP: 1-Methylpyrrolidin-2-on
- RT: Raumtemperatur
- TBTMG: N"-tert-Butyl-N,N,N',N'-tetramethyl-guanidin
- THF: Tetrahydrofuran

### Beispiel 1: N-[5-Methansulfonyl-4-(4-methansulfonyl-3-pentafluorsulfanyl-phenoxy)-2-methyl-benzoyl]-guanidin

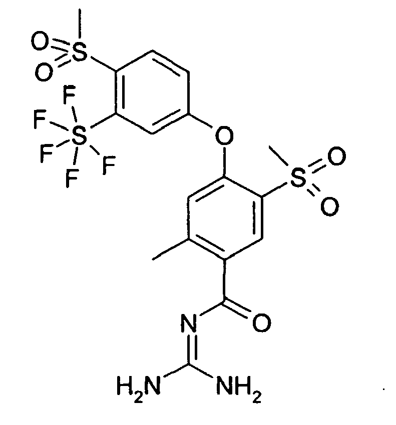

### a) 3-Pentafluorsulfanyl-phenol

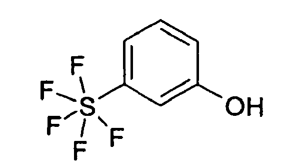

5.0 g 3-Pentafluorsulfanyl-anilin wurden in 50 ml einer 35% wäßrigen H₂SO₄-Lösung suspendiert. Bei 0°C wurde dann eine Lösung von 1.57 g NaNO₂ in 5 ml Wasser innerhalb 10 Minuten zugetropft. 40 Minuten lang wurde bei 0°C nachgerührt. Zu dieser Suspension wurde dann eine auf 0°C gekühlte Lösung von 8.56 g Cu(NO₃)₂ in 50 ml Wasser zugegeben. Direkt danach wurde noch 3.26 g Cu₂O zugegeben, wobei deutliche Gasentwicklung zu beobachten war. 3 mal wurde mit je 100 ml CH₂Cl₂ extrahiert, die org. Phase mit 100 ml einer gesättigten wäßrigen NaCl-Lsg. gewaschen, über MgSO₄ getrocknet und das Solvens im Vakuum entfernt. Chromatographie mit DIP an einer kurzen Kieselgelsäule lieferte 3.5 g des Phenols als farbloses Öl.

| | |
|---|---|
| R_{f} (EE/HEP 1:10) = 0.15 | MS (EI) : 220 |

### b) 5-Methansulfonyl-2-methyl-4-(3-pentafluorsulfanyl-phenoxy)-benzoesäuremethylester

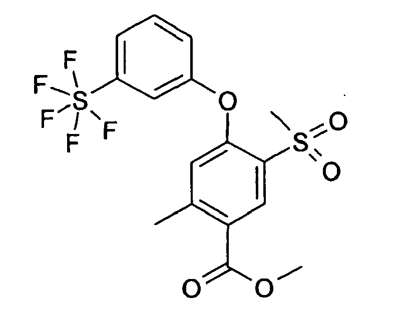

600 mg 4-Fluor-5-methansulfonyl-2-methyl-benzoesäure-methylester, 700 mg 3-Pentafluorsulfanyl-phenol sowie 1.6 g Cs₂CO₃ wurden in 4 ml wasserfreiem DMF 3 h bei 100°C gerührt. Dann wurde auf RT abgekühlt, mit 100 ml EE verdünnt und 3 mal mit je 20 ml Wasser gewaschen. Über MgSO₄ wurde getrocknet, das Solvens im Vakuum entfernt und der Rückstand an Kieselgel mit DIP chromatographiert. Man erhielt 300 mg eines farblosen Öls.

| | |
|---|---|
| R_{f} (DIP) = 0.27 | MS (ES⁺) : 446 |

### c) 5-Methansulfonyl-4-(4-nitro-3-pentafluorsulfanyl-phenoxy)-2-methyl-benzoesäuremethylester

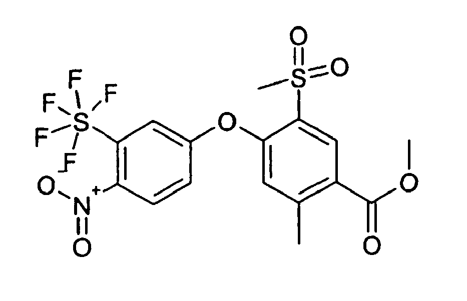

3.50 g 5-Methansulfonyl-2-methyl-4-(3-pentafluorsulfanyl-phenoxy)-benzoesäuremethylester wurde bei -40°C in 100 ml 90% HNO₃ gelöst. 10 Minuten wurde bei dieser Temperatur gerührt und dann das Reaktionsgemisch auf 800 g Eis gegossen. Dies Gemisch wurde 10 Minuten gerührt und anschließend das Produkt abgesaugt. Man erhielt 3.89 g eines blassgelben Feststoffs, mp145-148°C (unter Zersetzung).
R_{f} (DIP) = 0.09

### d) 4-(4-Amino-3-pentafluorsulfanyl-phenoxy)-5-methansulfonyl-2-methyl-benzoesäuremethylester

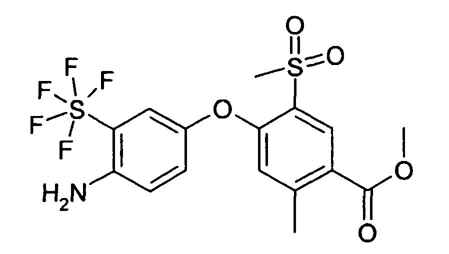

3.80 g 5-Methansulfonyl-4-(4-nitro-3-pentafluorsulfanyl-phenoxy)-2-methylbenzoesäure-methylester wurden in 30 ml HOAc sowie 30 ml MeOH gelöst, mit 200 mg Pd/C (10%) versetzt und 24 h bei 6 bar Wasserstoffdruck hydriert. Da die Umsetzung noch nicht vollständig war, wurden weitere 300 mg Pd/C (10%), 30 ml HOAc und 30 ml MeOH zugegeben und weitere 24 h bei 6 bar Wasserstoffdruck hydriert. Anschließend wurde der Katalysator abfiltriert und die Solventien im Vakuum entfernt. Man erhielt 3.4 g eines hellgrauen Feststoffs, mp 175°C (unter Zersetzung).
R_{f} (MTB) = 0.44

### e) 4-(4-Chlorsulfonyl-3-pentafluorsulfanyl-phenoxy)-5-methansulfonyl-2-methylbenzoesäure-methylester

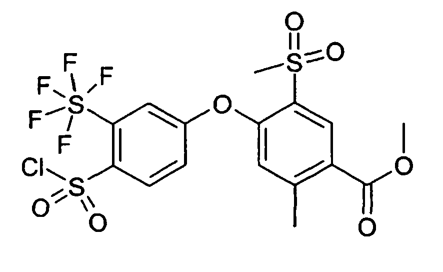

3.4 g 4-(4-Amino-3-pentafluorsulfanyl-phenoxy)-5-methansulfonyl-2-methylbenzoesäure- methylester wurden in 30 ml HOAc gelöst, mit 30 g Eis und anschließend mit 30 ml einer gesättigten wäßrigen HCl-Lösung versetzt. Zu dieser Lösung wurde bei 0°C eine Lösung von 0.56 g NaNO₂ in 5 ml Wasser innerhalb von 5 Minuten zugetropft. 10 Minuten wurde bei 0°C gerührt. Diese Lösung wurde dann portionsweise zu einer 0°C kalten Suspension aus 12.4 mg CuCl und 125.6 mg CuCl₂ (Dihydrat) in 100 ml einer mit SO₂ gesättigten HOAc addiert. 2 h wurde bei RT gerührt, dann mit 300 ml Wasser verdünnt und 3 mal mit je 200 ml EE extrahiert. Über MgSO4 wurde getrocknet und das Solvens im Vakuum entfernt. Man erhielt 3.5 g eines viskosen Öls, das ohne Reinigung weiter umgesetzt wurde.

### f) Natrium-4-(2-methansulfonyl-4-methoxycarbonyl-5-methyl-phenoxy)-2-pentafluorsulfanyl-benzolsulfinat

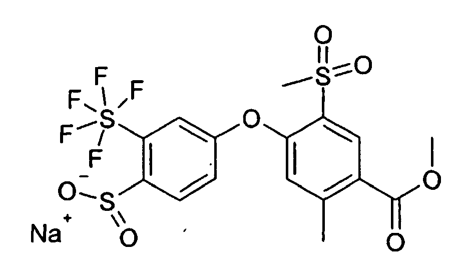

3.5 g 4-(4-Chlorsulfonyl-3-pentafluorsulfanyl-phenoxy)-5-methansutfonyl-2-methylbenzoesäure-methylester wurden portionsweise zu einer 70°C warmen Lösung von 8.10 g Na₂SO₃ in 75 ml Wasser addiert und dabei mit 10 molarer wässriger NaOH-Lösung der pH bei etwa pH=10 gehalten. Anschließend wird 45 Minuten bei 70°C nachgerührt, dann läßt man abkühlen und stellt mit wäßriger HCl-Lösung den pH auf pH=2. 3 mal wurde mit je 200 ml EE extrahiert. Über MgSO₄ wurde getrocknet und das Solvens im Vakuum entfernt. Der Rückstand wurde in 100 ml Wasser suspendiert, mit einer 2 molaren wäßrigen NaOH-Lösung auf pH=10 gestellt und die flüchtigen Bestandteile im Vakuum entfernt. Anschließend wurde zunächst 2 mal mit je 100 ml Toluol, dann mit 100 ml wasserfreiem DMF co-evaporiert und der Rückstand (3.0 g) ohne Reinigung weiter umgesetzt.

### g) 5-Methansulfonyl-4-(4-methansulfonyl-3-pentafluorsulfanyl-phenoxy)-2-methylbenzoesäure-methylester und 5-Methansulfonyl-4-(4-methoxysulfinyl-3-pentafluorsulfanyl-phenoxy)-2-methylbenzoesäure-methylester

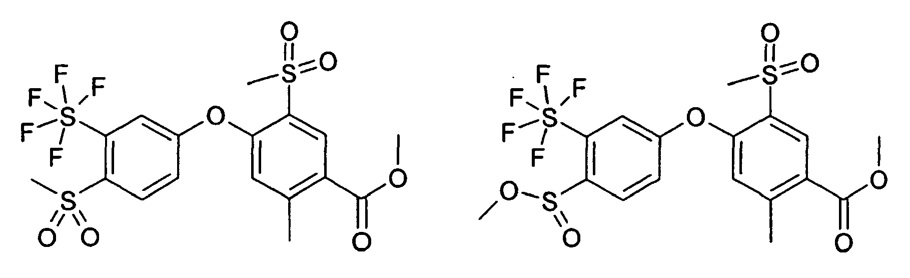

3.0 g Natrium-4-(2-methansulfonyl-4-methoxycarbonyl-5-methyl-phenoxy)-2-pentafluorsulfanyl-benzolsulfinat wurden in 100 ml wasserfreiem DMF gelöst, 4.0 g CH₃I hinzugegeben und 9 h bei 45°C gerührt. Anschließend wurde das Reaktionsgemisch 2 Tage bei RT stehen gelassen. Dann wurde das Solvens im Vakuum entfernt und der Rückstand mit 100 ml Wasser sowie 100 ml EE aufgenommen. Dann wurden 50 ml einer 5% wäßrigen NaHSO₄-Lösung zugegeben und die Phasen getrennt. Anschließend wurde 3 mal mit je 100 ml EE extrahiert. Über MgSO₄ wurde getrocknet, das Solvens im Vakuum entfernt und der Rückstand an Kieselgel mit MTB/DIP 1:1 chromatographiert. Man erhielt 0.59 g 5-Methansulfonyl-4-(4-methansulfonyl-3-pentafluorsulfanyl-phenoxy)-2-methyl-benzoesäure-methylester und 0.49 g 5-Methansulfonyl-4-(4-methoxysulfinyl-3-pentafluorsulfanyl-phenoxy)-2-methyl-benzoesäure-methylester.
Rf (MTB/DIP 1:1) = 0.13: 5-Methansulfonyl-4-(4-methansulfonyl-3-pentafluorsulfanylphenoxy)-2-methyl-benzoesäure-methylester
Rf (MTB/DIP 1:1) = 0.32: 5-Methansulfonyl-4-(4-methoxysulfinyl-3-pentafluorsulfanylphenoxy)-2-methyl-benzoesäure-methylester

### h) N-[5-Methansulfonyl-4-(4-methansulfonyl-3-pentafluorsulfanyl-phenoxy)-2-methylbenzoyl]-guanidin

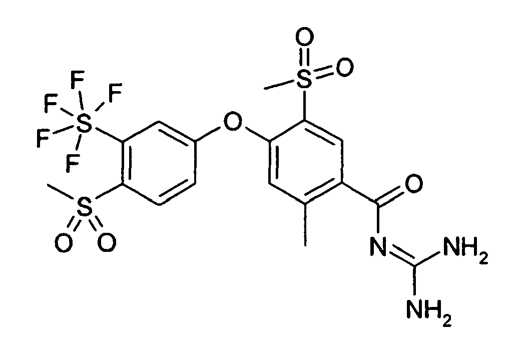

546 mg Guanidinium-chlorid sowie 535 mg KOtBu wurden in 20 ml wasserfreiem DMF 30 Minuten bei RT gerührt. Dann wurden 500 mg 5-Methansulfonyl-4-(4-methansulfonyl-3-pentafluorsulfanyl-phenoxy)-2-methyl-benzoesäure-methylester zugegeben und 16 Stunden bei RT stehen gelassen. Das Reaktionsgemisch wurde auf 20 ml Wasser gegossen, mit wäßriger HCl-Lösung auf pH = 8 gestellt und 3 mal mit je 50 ml EE extrahiert. Die vereinigten EE-Phasen wurden dann mit 20 ml einer 5% wäßrigen NaHCO₃-Lösung gewaschen, über MgSO₄ getrocknet und das Solvens im Vakuum entfernt. Man erhielt 420 mg eines amorphen Feststoffs.

| | |
|---|---|
| Rf (EE/MeOH 10:1) = 0.29 | MS (ES⁺) : 551 |

### k) N-[5-Methansulfonyl-4-(4-methansulfonyl-3-pentafluorsulfanyl-phenoxy)-2-methylbenzoyl]-guanidin, Hydrochlorid

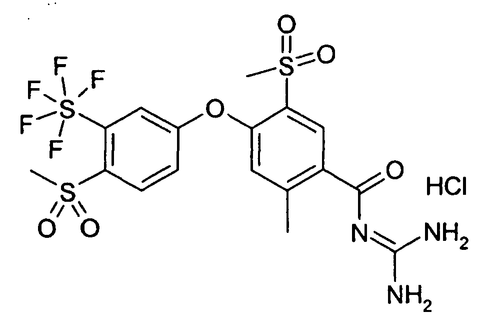

400 mg N-[5-Methansulfonyl-4-(4-methansulfonyl-3-pentafluorsulfanyl-phenoxy)-2-methyl-benzoyl]-guanidin wurden in verdünnter wäßriger HCl-Lösung gelöst und dann die flüchtigen Bestandteile im Vakuum entfernt. Der Rückstand wurde aus Wasser umkristallisiert und man erhielt 200 mg eines hellgrauen Feststoffs, mp 275°C.

### Bestimmung der NHE-Hemmung

Die Hemmkonzentration IC₅₀ für die NHE-1 Hemmung wurde wie folgt bestimmt:

IC₅₀ für die NHE-1 Hemmung wurde bestimmt in einem FLIPR-Assay mittels Messung der pHᵢ-Erholung in transfizierten Zelllinien, die den humanen NHE-1 exprimieren.
Der Assay wurde im FLIPR (Fluorometric imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 weist als Folge von Mutagenese und anschließender Selektion keine endogene NHE-Aktivität auf), wurden am Vortag mit einer Dichte von ~25.000 Zellen / Well ausgesät. Das Wachstumsmedium der transfizierten Zellen (Iscove +10 % fötales Kälberserum) enthielt zusätzlich G418 als Selektionsantibiotikum, um die Anwesenheit der transfizierten Sequenzen sicherzustellen.

Der eigentliche Assay begann mit der Entfernung des Wachstumsmediums und Zugabe von 100 µl /Well Beladungspuffer (5 µM BCECF-AM [2',7'-Bis-(Carboxyethyl)-5-(und-6)-Carboxyfluorescein, Acetoxymethyl ester] in 20 mM NH₄Cl, 115 mM Cholinchlorid, 1 mM MgCl₂, 1 mM CaCl₂, 5 mM KCl, 20 mM HEPES, 5 mM Glucose; pH 7,4 [mit KOH eingestellt]). Die Zellen wurden dann 20 Minuten bei 37°C inkubiert. Diese Inkubation führte zur Beladung der Zellen mit dem fluoreszierenden Farbstoff, dessen Fluoreszenzintensität vom pHi abhängt, und mit NH₄Cl, was zu einer leichten Alkalinisierung der Zellen führte.
Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.

Nach dieser 20-minütigen Inkubation wurde der Beladungspuffer, der NH₄Cl und freies BCECF-AM enthielt, durch dreimaliges Waschen im Zellwasher (Tecan Columbus) mit jeweils 400 µl Waschpuffer (133,8 mM Cholinchlorid, 4,7 mM KCI, 1,25 mM MgCl₂, 1,25 mM CaCl₂, 0,97 mM K₂HPO₄, 0,23 mM KH₂PO₄, 5 mM HEPES, 5 mM Glucose; pH 7,4 [mit KOH eingestellt]) entfernt. Das in den Wells verbleibende Restvolumen betrug 90 µl (50 - 125 µl möglich). Dieser Waschschritt entfernte das freie BCECF-AM und führte als Folge der Entfernung der externen NH₄⁺-lonen zu einer intrazellulären Ansäuerung (∼ pHi 6.3 - 6.4).
Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wurde, führte der Waschprozess dazu, dass intrazellulär H⁺ zurückblieb, was die Ursache für die intrazelluläre Ansäuerung war. Diese kann letztlich zum Zelltod führen, wenn sie lang genug anhält. An dieser Stelle war es wichtig, dass der Waschpuffer natriumfrei (<1 mM) war, da extrazelluläre Natrium-lonen zu einer augenblicklichen Erholung des pHᵢ durch die Aktivität der klonierten NHE-Isoformen führen würde.
Es war ebenfalls wichtig, dass alle verwendeten Puffer (Beladungspuffer, Waschpuffer, Recoverypuffer) keine HCO₃⁻-lonen enthielten, da die Anwesenheit von Bicarbonat zur Aktivierung störender bicarbonatabhängiger pHᵢ-Regulationssysteme führen würde, die in der parentalen LAP-1 Zelllinie enthalten sind.

Die Mikrotiterplatten mit den angesäuerten Zellen wurden dann (bis zu 20 Minuten nach der Ansäuerung) zum FLIPR transferiert. Im FLIPR wurde der intrazelluläre Fluoreszenzfarbstoff durch Licht mit einer Wellenlänge von 488 nm, das von einem Argon-Laser erzeugt wurde, angeregt, und die Messparameter (Laserleistung, Belichtungszeit und Blende der im FLIPR eingebauten CCD-Kamera) wurden so gewählt, dass das durchschnittliche Fluoreszenzsignal pro Well zwischen 30000 und 35000 relativen Fluoreszenzeinheiten lag.

Die eigentliche Messung im FLIPR begann damit, dass Software-gesteuert alle zwei Sekunden eine Aufnahme mit der CCD-Kamera gemacht wurde. Nach zehn Sekunden wurde die Erholung des intrazellulären pH's durch Zugabe von 90 µl Recoverypuffer (133,8 mM NaCl, 4,7 mM KCI, 1,25 mM MgCl₂, 1,25 mM CaCl₂, 0,97 mM K₂HP0₄, 0,23 mM KH₂PO₄, 10 mM HEPES, 5 mM Glucose; pH 7.4 [mit NaOH eingestellt]) mittels des im FLIPR eingebauten 96-Well-Pipettierers eingeleitet.
Als Positivkontrollen (100 % NHE-Aktivität) dienten Wells, denen reiner Recoverypuffer zugegeben wurde, Negativkontrollen (0 % NHE-Aktivität) erhielten Waschpuffer. In allen anderen Wells wurde Recoverypuffer mit der zweifach konzentrierten Testsubstanz hinzugegeben. Die Messung im FLIPR endete nach 60 Messpunkten (zwei Minuten).

Die Rohdaten werden in das Programm ActivityBase exportiert. Mit diesem Programm werden zunächst die NHE-Aktivitäten für jede getestete Substanzkonzentration und daraus die IC₅₀-Werte für die Substanzen berechnet. Da der Verlauf der pHᵢ-Erholung nicht während des ganzen Experiments linear war, sondern am Ende aufgrund abnehmender NHE-Aktivität bei höheren pHᵢ-Werten abfiel, war es wichtig, für die Auswertung der Messung den Teil auszuwählen, in dem die Fluoreszenzzunahme der Positivkontrollen linear war.

| Beispiel | NHE1-Hemmung IC₅₀ [nM] |
|---|---|
| 1 | 33 |

## Patentansprüche

1. Pentafluorsulfanyl-benzoylguanidin-e der Formel I 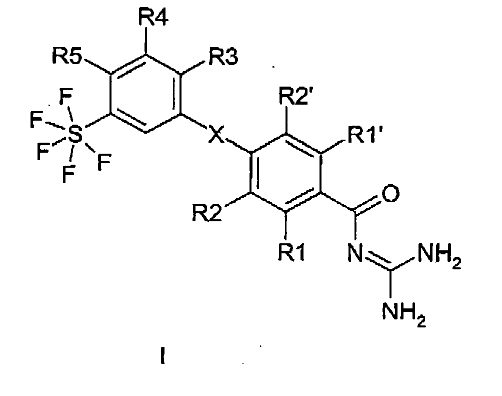 worin bedeuten
R1 und R1' unabhängig voneinander Wasserstoff, Methyl, F, Cl, -CF₃ oder -O-CH₂-CF₃;
R2 und R2' unabhängig voneinander Wasserstoff, F, Cl, -SO₂CH₃, -SO₂-CF₃, -CF₃ oder Methyl;
R3 Wasserstoff, F, Cl oder Methyl;
R4 Wasserstoff, F, Cl oder Methyl;
R5 -SO₂CH₃;
X O;
sowie deren pharmazeutisch verträgliche Salze.

2. Verbindungen der Formel I nach Anspruch 1 ausgewählt aus:
N-[5-Methansulfonyl-4-(4-methansulfonyl-3-pentafluorsulfanyl-phenoxy)-2-methylbenzoyl]-guanidin
und dessen pharmazeutisch verträgliche Salze.

3. Verfahren zur Herstellung einer Verbindung der Formel I nach einem der Ansprüche 1 oder 2 und/oder deren pharmazeutisch verträgliche Salze, **dadurch gekennzeichnet, dass** man eine Verbindung der Formel VI, 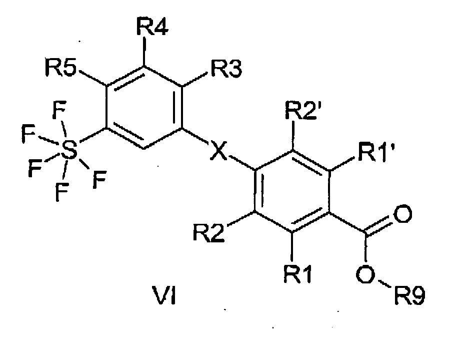 wobei R1, R1', R2, R2', R3, R4, R5 und X die oben genannten Bedeutungen haben und R9 Wasserstoff oder Alkyl mit 1, 2, 3 oder 4 C-Atomen ist, mit Guanidin umsetzt.

4. Verbindung der Formel I und/oder deren pharmazeutisch verträgliche Salze nach einem der Ansprüche 1 oder 2 zur Verwendung als Medikament.

5. Verwendung einer Verbindung der Formel I und/ader deren pharmazeutisch verträgliche Salze nach einem oder mehreren der Ansprüche 1 oder 2 zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrahlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasie, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zum Einsatz bei Bypassoperationen, zum Einsatz bei Wiederbelebung nach Herzstillstand, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyreotoxikose oder zur Herstellung eines Diagnostikums.

6. Verwendung einer Verbindung der Formel I und/oder deren pharmazeutisch verträgliche Salze nach einem der Ansprüche 1 oder 2 in Kombination mit anderen Arzneimitteln oder Wirkstoffen zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasie, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zum Einsatz bei Bypassoperationen, zum Einsatz bei Wiederbelebung nach Herzstillstand, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyreotoxikose oder zur Herstellung eines Diagnostikums.

7. Verwendung einer Verbindung der Formel I und/oder deren pharmazeutisch verträgliche Salze nach Anspruch 6 in der Kombination mit cardiotoxischen und cytotoxischen Arzneimitteln oder Wirkstoffen zur Herstellung eines Medikaments mit verminderten cardiotoxischen und cytotoxischen Eigenschaften.

8. Verwendung einer Verbindung der Formel I und/oder deren pharmazeutisch verträgliche Salze allein oder in Kombination mit anderen Arzneimitteln oder Wirkstoffen nach Anspruch 5 und/oder 6 zur Herstellung eines Medikaments zur Behandlung oder Prophylaxe von akuten oder chronischen Schäden, Erkrankungen oder indirekten Folgeerkrankungen von Organen und Geweben, die durch Ischämie- oder durch Reperfusionsereignisse verursacht werden.

9. Verwendung einer Verbindung der Formel I und/oder deren pharmazeutisch verträgliche Salze allein oder in Kombination mit anderen Arzneimitteln oder Wirkstoffen nach Anspruch 5 und/oder 6 zur Herstellung eines Medikaments zur Behandlung lebensbedrohlichen Kammerflimmerns des Herzens.

10. Verwendung einer Verbindung der Formel I und/oder deren pharmazeutisch verträgliche Salze allein oder in Kombination mit anderen Arzneimitteln oder Wirkstoffen nach Anspruch 5 und/oder 6 zur Herstellung eines Medikaments zur Behandlung oder zur Prophylaxe der Metastasierung.

11. Verwendung einer Verbindung der Formen I und/oder deren pharmazeutisch verträgliche Salze allein oder in Kombination mit anderen Arzneimitteln oder Wirkstoffen nach Anspruch 5 und/oder 6 zur Herstellung eines Medikaments zur Behandlung oder Prophylaxe fibrotischer Erkrankungen des Herzens, der Herzinsuffizienz oder des Congestive Heart failure.

12. Heilmittel für die humane, veterinäre und/oder phytoprotektive Anwendung enthaltend eine wirksame Menge einer Verbindung der Formel I und/oder deren pharmazeutische verträgliche Salze nach einem der Ansprüche 1 oder 2, zusammen mit pharmazeutisch annehmbaren Träger- und Zusatzstoffen.

13. Heilmittel für die humane, veterinäre oder phytoprotektive Anwendung enthaltend eine wirksame Menge einer Verbindung der Formel I und/oder deren pharmazeutisch verträgliche Salze nach einem der Ansprüche 1 oder 2, zusammen mit pharmazeutisch annehmbaren Träger- und Zusatzstoffen in Kombination mit anderen pharmakologischen Wirkstoffen oder Arzneimitteln.

## Claims

1. A pentafluorosulfanylbenzoylguanidine of the formula I 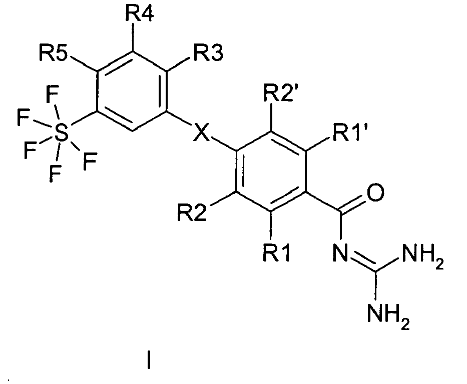 in which the meanings are
R1 and R1' independently of one another hydrogen, methyl, F, Cl, -CF₃ or -O-CH₂-CF₃;
R2 and R2' independently of one another hydrogen, F, Cl, -SO₂CH₃, -SO₂-CF₃, -CF₃ or methyl;
R3 hydrogen, F, Cl or methyl;
R4 hydrogen, F, Cl or methyl;
R5 -SO₂CH₃;
X O;
and the pharmaceutically acceptable salts thereof.

2. A compound of the formula I as claimed in claim 1 selected from:
N-[5-methanesulfonyl-4-(4-methanesulfonyl-3-pentafluorosulfanylphenoxy)-2-methyl-benzoyl]guanidine
and its pharmaceutically acceptable salts.

3. A process for preparing a compound of the formula I as claimed in either of claims 1 and 2 and/or the pharmaceutically acceptable salts thereof, which comprises reacting a compound of the formula VI 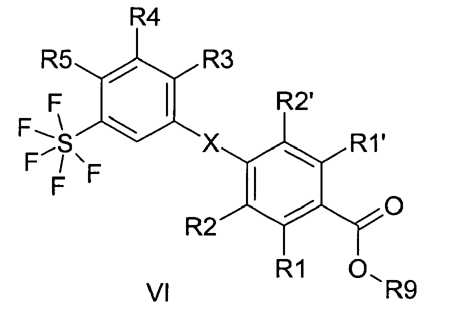 where R1, R1', R2, R2', R3, R4, R5 and X have the abovementioned meanings, and R9 is hydrogen or alkyl having 1, 2, 3 or 4 carbon atoms with guanidine.

4. The compound of the formula I and/or the pharmaceutically acceptable salts thereof as claimed in either of claims 1 and 2 for use as medicament.

5. The use of a compound of the formula I and/or the pharmaceutically acceptable salts thereof as claimed in one or more of claims 1 and 2 for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, epilepsy or centrally induced convulsions, of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria or of coccidiosis in poultry, and for use for surgical operations and organ transplantations, for preserving and storing transplants for surgical procedures, for use for bypass surgery, for use for resuscitation after cardiac arrest, for preventing age-related tissue change, for producing a medicament directed against aging or for prolonging life, for the treatment and reduction of the cardiotoxic effects in thyrotoxicosis or for producing a diagnostic aid.

6. The use of a compound of the formula I and/or the pharmaceutically acceptable salts thereof as claimed in either of claims 1 and 2 in combination with other medicaments or active ingredients for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, epilepsy or centrally induced convulsions, of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria or of coccidiosis in poultry, and for use for surgical operations and organ transplantations, for preserving and storing transplants for surgical procedures, for use for bypass surgery, for use for resuscitation after cardiac arrest, for preventing age-related tissue change, for producing a medicament directed against aging or for prolonging life, for the treatment and reduction of the cardiotoxic effects in thyrotoxicosis or for producing a diagnostic aid.

7. The use of a compound of the formula I and/or the pharmaceutically acceptable salts thereof as claimed in claim 6 in combination with cardiotoxic and cytotoxic medicaments or active ingredients for producing a medicament with reduced cardiotoxic and cytotoxic properties.

8. The use of a compound of the formula I and/or the pharmaceutically acceptable salts thereof alone or in combination with other medicaments or active ingredients as claimed in claim 5 and/or 6 for producing a medicament for the treatment or prophylaxis of acute or chronic damage, disorders or indirect sequelae of organs and tissues caused by ischemic or reperfusion events.

9. The use of a compound of the formula I and/or the pharmaceutically acceptable salts thereof alone or in combination with other medicaments or active ingredients as claimed in claim 5 and/or 6 for producing a medicament for the treatment of life-threatening cardiac ventricular fibrillation.

10. The use of a compound of the formula I and/or the pharmaceutically acceptable salts thereof alone or in combination with other medicaments or active ingredients as claimed in claim 5 and/or 6 for producing a medicament for the treatment or prophylaxis of metastasis.

11. The use of a compound of the formula I and/or the pharmaceutically acceptable salts thereof alone or in combination with other medicaments or active ingredients as claimed in claim 5 and/or 6 for producing a medicament for the treatment or prophylaxis of fibrotic disorders of the heart, of heart failure or of congestive heart failure.

12. A medicine for human, veterinary and/or phytoprotective use comprising an effective amount of a compound of the formula I and/or the pharmaceutically acceptable salts thereof as claimed in either of claims 1 and 2, together with pharmaceutically acceptable carriers and additives.

13. A medicine for human, veterinary or phytoprotective use comprising an effective amount of a compound of the formula I and/or the pharmaceutically acceptable salts thereof as claimed in either of claims 1 and 2, together with pharmaceutically acceptable carriers and additives in combination with other pharmacological active ingredients or medicaments.

## Revendications

1. Pentafluorosulfanyl-benzoylguanidines de formule I 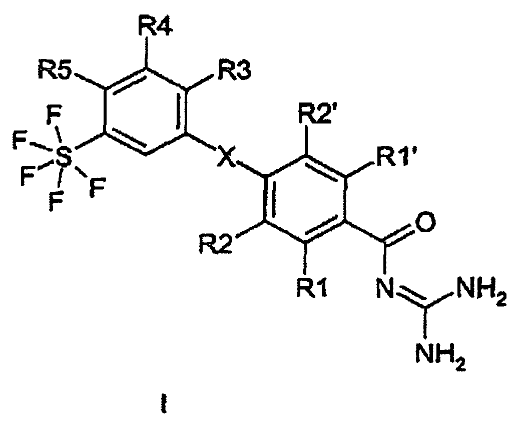 dans laquelle
R1 et R1' représentent, indépendamment l'un de l'autre, un atome d'hydrogène, le groupe méthyle, F, Cl, -CF₃ ou -O-CH₂-CF₃ ;
R2 et R2' représentent, indépendamment l'un de l'autre, un atome d'hydrogène, F, Cl, -SO₂CH₃, -SO₂CF₃, -CF₃ ou le groupe méthyle ;
R3 représente un atome d'hydrogène, F, Cl ou le groupe méthyle ;
R4 représente un atome d'hydrogène, F, Cl ou le groupe méthyle ;
R5 représente -SO₂CH₃ ;
X représente O ;
ainsi que leurs sels pharmaceutiquement acceptables.

2. Composés de formule I selon la revendication 1, choisis parmi :
la N-[5-méthanesulfonyl-4-(4-méthanesulfonyl-3-pentafluorosulfanyl-phénoxy)-2-méthyl-benzoyl]-guanidine et ses sels pharmaceutiquement acceptables.

3. Procédé pour la préparation d'un composé de formule I selon la revendication 1 ou 2 et/ou de ses sels pharmaceutiquement acceptables, **caractérisé en ce qu'**on fait réagir avec de la guanidine un composé de formule VI, 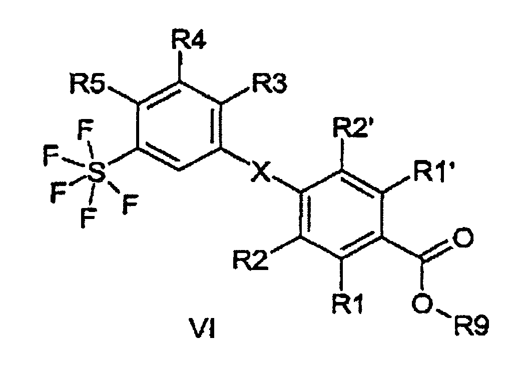 dans laquelle R1, R1', R2, R2', R3, R4, R5 et X ont les significations données plus haut et R9 est un atome d'hydrogène ou un groupe alkyle ayant 1, 2, 3 ou 4 atomes de carbone.

4. Composé de formule I et/ou sels pharmaceutiquement acceptables d'un tel composé, selon la revendication 1 ou 2, pour utilisation en tant que médicament.

5. Utilisation d'un composé de formule I et/ou de ses sels pharmaceutiquement acceptables selon une ou plusieurs des revendications 1 et 2, pour la fabrication d'un médicament destiné au traitement ou à la prophylaxie de troubles, maladies ou maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, de l'hypertonie essentielle, de maladies du système nerveux central, de maladies qui résultent d'une hyperexcitabilité du système nerveux central, de l'épilepsie ou des crises déclenchées par le système nerveux central, de maladies du système nerveux central, en particulier d'états anxieux, de dépressions ou de psychoses, au traitement ou à la prophylaxie du diabète sucré non insulinodépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la *congestive heart failure,* de maladies inflammatoires aiguës ou chroniques, de maladies qui sont provoquées par des protozoaires, du paludisme et de la coccidiose du poulet et à l'utilisation dons des interventions chirurgicales et des transplantations d'organes, pour la conservation et le stockage de transplants pour des interventions chirurgicales, à l'utilisation dans des opérations de pontage, à l'utilisation dans la réanimation après arrêt du coeur, pour empêcher une altération tissulaire due à l'âge, pour la fabrication d'un médicament dirigé contre le vieillissement ou pour le prolongement de la vie, au traitement et à l'abaissement des effets cardiotoxiques dans la thyréotoxicose ou à la fabrication d'un agent de diagnostic.

6. Utilisation d'un composé de formule I et/ou de ses sels pharmaceutiquement acceptables selon la revendication 1 ou 2, en association avec d'autres médicaments ou principes actifs pour la fabrication d'un médicament destiné au traitement ou à la prophylaxie de troubles, maladies ou maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, de l'hypertonie essentielle, de maladies du système nerveux central, de maladies qui résultent d'une hyperexcitabilité du système nerveux central, telles que l'épilepsie ou des crises déclenchées par le système nerveux central, de maladies du système nerveux central, en particulier d'états anxieux, de dépressions ou de psychoses, au traitement ou à la prophylaxie du diabète sucré non insulinodépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la *congestive heart failure*, de maladies inflammatoires aiguës ou chroniques, de maladies qui sont provoquées par des protozoaires, du paludisme et de la coccidiose du poulet et à l'utilisation dons des interventions chirurgicales et des transplantations d'organes, pour la conservation et le stockage de transplants pour des interventions chirurgicales, à l'utilisation dans des opérations de pontage, à l'utilisation dans la réanimation après arrêt du coeur, pour empêcher une altération tissulaire due à l'âge, pour la fabrication d'un médicament dirigé contre le vieillissement ou pour le prolongement de la vie, au traitement et à l'abaissement des effets cardiotoxiques dans la thyréotoxicose ou à la fabrication d'un agent de diagnostic.

7. Utilisation d'un composé de formule I et/ou de ses sels pharmaceutiquement acceptables selon la revendication 6, en association avec des principes actifs ou des médicaments cardiotoxiques et cytotoxiques, pour la fabrication d'un médicament à propriétés cardiotoxiques et cytotoxiques réduites.

8. Utilisation d'un composé de formule I et/ou de ses sels pharmaceutiquement acceptables, seul(s) ou en association avec d'autres principes actifs ou médicaments selon la revendication 5 et/ou la revendication 6, pour la fabrication d'un médicament destiné au traitement ou à la prophylaxie de troubles, de maladies ou de maladies secondaires indirectes, chroniques ou aigus, d'organes et de tissus, qui sont provoqués par des incidents ischémiques ou par des incidents de reperfusion.

9. Utilisation d'un composé de formule I et/ou de ses sels pharmaceutiquement acceptables, seul(s) ou en association avec d'autres principes actifs ou médicaments selon la revendication 5 et/ou la revendication 6, pour la fabrication d'un médicament destiné au traitement de la fibrillation ventriculaire du coeur, mettant la vie en danger.

10. Utilisation d'un composé de formule I et/ou de ses sels pharmaceutiquement acceptables, seul(s) ou en association avec d'autres principes actifs ou médicaments selon la revendication 5 et/ou la revendication 6, pour la fabrication d'un médicament destiné au traitement ou à la prophylaxie de la dissémination de métastases.

11. Utilisation d'un composé de formule I et/ou de ses sels pharmaceutiquement acceptables, seul(s) ou en association avec d'autres principes actifs ou médicaments selon la revendication 5 et/ou la revendication 6, pour la fabrication d'un médicament destiné au traitement ou à la prophylaxie de maladies fibreuses du coeur, de l'insuffisance cardiaque ou de la *congestive heart failure.*

12. Médicament pour l'utilisation humaine, vétérinaire et/ou phytosanitaire, contenant une quantité efficace d'un composé de formule I et/ou de ses sels pharmaceutiquement acceptables selon la revendication 1 ou 2, conjointement avec des véhicules et additifs pharmaceutiquement acceptables.

13. Médicament pour l'utilisation humaine, vétérinaire ou phytosanitaire, contenant une quantité efficace d'un composé de formule I et/ou de ses sels pharmaceutiquement acceptables selon la revendication 1 ou 2, conjointement avec des véhicules et additifs pharmaceutiquement acceptables, en association avec d'autres médicaments ou principes actifs pharmacologiques.
